# DEMANDE DE BREVET EUROPEEN

(11) **EP 1 690 521 A1**
(43) Date de publication de la demande: **16.08.2006**
(21) Numéro de dépôt: 06100322.4
(22) Date de dépôt: 13.01.2006
(51) Int. Cl.: A61K 8/02

(54) **Article cosmétique de démaquillage**

(30) Priorité: 09.02.2005 FR 0550386
(71) Demandeur: L'Oreal-D.I.P.I., 75008 Paris (FR)
(72) Inventeur: SIMON, Pascal, 94320, THIAIS (FR)
(74) Mandataire: Rasson, Catherine

(57) **Abrégé**

L'invention se rapporte à un article comportant (A) un support insoluble dans l'eau, constitué d'une mousse hydrophile de polyuréthanne, et (B) une composition cosmétique imprégnée sur le support et constituée d'une phase aqueuse et d'une phase huileuse distinctes en un rapport pondéral allant de 25/75 à 90/10.

La mousse peut être notamment une mousse de polyuréthanne à cellules ouvertes..

Cet article peut être utilisée en particulier pour le nettoyage et/ou le démaquillage de la peau du visage et/ou du corps, ainsi que pour le démaquillage des yeux.

## Description

L'invention se rapporte à un article comportant au moins un support insoluble dans l'eau et une composition cosmétique comportant deux phases distinctes non miscibles, ainsi qu'aux utilisations dudit article dans le domaine cosmétique, en particulier pour le nettoyage et/ou le démaquillage de la peau humaine, plus spécialement de la peau du visage, des lèvres et/ou des yeux.

Les articles tels que les lingettes cosmétiques nettoyantes et démaquillantes sont utilisés couramment et sont appréciés pour leur côté pratique car ils sont jetables, ils sont imprégnés de la quantité nécessaire et suffisante de produit nettoyant ou démaquillant, et ils évitent la manipulation et le transport de flacons contenant les lotions ou laits. Ces articles sont généralement constitués d'un support en une matière d'origine naturelle ou synthétique, qui est de préférence un non tissé, ledit support étant imprégné d'une composition adaptée au but recherché, par exemple le nettoyage ou le démaquillage de la peau, ou encore le soin de la peau.

Par ailleurs, il est connu d'utiliser comme composition démaquillante, des compositions ayant deux phases distinctes, une phase aqueuse et une phase huileuse non miscibles, comme c'est décrit par exemple dans les documents FR-A-2,847,468, FR-A-2,753,090 et EP-A-0370856. Les compositions de ce type, constituées de deux phases distinctes, notamment d'une phase aqueuse et d'une phase huileuse distinctes et non émulsionnées l'une dans l'autre au repos, sont généralement désignées sous le terme de "composition biphase". Elles se distinguent des émulsions par le fait qu'au repos, les deux phases sont distinctes au lieu d'être émulsionnées l'une dans l'autre et qu'elles ne forment une dispersion H/E que lorsqu'elles sont agitées. L'utilisation de ces compositions biphases nécessite donc une agitation préalable afin de former extemporanément une dispersion, celle-ci devant être de qualité et de stabilité suffisantes pour permettre une application homogène des deux phases sur la peau ou toute autre matière kératinique sur laquelle elle est appliquée. Au repos, lesdites phases doivent se séparer rapidement et retrouver leur état initial, ce phénomène étant plus connu sous le terme de "déphasage".

Ce type de démaquillant permet de démaquiller tous les types de maquillage, waterproof et non waterproof, et il a l'avantage de permettre un démaquillage aussi efficace qu'avec une huile, tout en ayant des propriétés cosmétiques intéressantes, contrairement aux huiles qui, elles, présentent des désagréments à l'utilisation. En effet, les compositions composées seulement d'huiles laissent un film résiduel gras sur la peau, alors qu'une composition biphase, une fois agitée, se transforme en une dispersion de gouttelettes d'huile dans la phase aqueuse, et ne laissent pas de film gras résiduel, tout en conservant une efficacité de démaquillage comparable à celle d'une composition ne comprenant que de l'huile.

Dans le but de combiner le côté pratique des lingettes et l'efficacité des démaquillants à deux phases distinctes, il paraît judicieux de vouloir imprégner ce type de démaquillant sur un non-tissé. L'imprégnation des compositions biphases sur un non-tissé ne pose pas de problèmes dans la mesure où la viscosité de la composition n'est pas élevée. De plus, le produit imprégné est très bien restitué, ce qui permet un bon démaquillage. Toutefois, la stabilité dans le temps d'un tel produit n'est pas suffisante, car, dans les conditionnements regroupant une pile de lingettes ou de tampons à démaquiller, on observe un phénomène de sédimentation avec le dépôt de la phase aqueuse au fond de la boîte ou de l'emballage contenant les lingettes, si bien qu'après un temps de conservation relativement court, d'une à deux semaines, les lingettes situées en haut de la pile sont davantage imprégnées d'huile que celles du bas et, de ce fait, chaque lingette n'est plus imprégnée de la même proportion de chaque phase selon son emplacement dans la pile, comme cela ressort du test présenté plus loin.

Ainsi, il subsiste le besoin de disposer d'un article de type lingette contenant une composition alliant efficacité et confort comme l'est une composition biphase, qui reste stable en composition, de sorte que plusieurs articles empilés dans un paquet restent imprégnés de la même manière au cours du temps.

La demanderesse a trouvé de manière surprenante que l'utilisation d'un support en mousse hydrophile en polyuréthanne permettait d'éviter ce phénomène de sédimentation tout en conservant les bonnes propriétés cosmétiques des lingettes en non-tissé (douceur, bonne disponibilité de la composition d'imprégnation à l'essuyage) et de la composition biphase.

La présente invention a donc pour objet un article comportant (A) un support insoluble dans l'eau, constitué d'une mousse hydrophile de polyuréthanne, et (B) une composition cosmétique imprégnée sur le support et constituée d'une phase aqueuse et d'une phase huileuse distinctes en un rapport pondéral allant de 25/75 à 90/10, les constituants de la phase huileuse ayant un point de fusion inférieur à 25°C.

La mousse hydrophile de polyuréthanne est formée avant imprégnation par la composition cosmétique, ce qui signifie que la composition cosmétique n'est donc pas imprégnée in situ lors de la fabrication de la mousse, comme c'est le cas par exemple pour l'émulsion décrite dans le document US-4,806,572, car une telle imprégnation in situ présente l'inconvénient d'obliger à avoir une quantité efficace de tensioactif émulsionnant et de ne pas permettre l'imprégnation d'une composition biphase.

Par ailleurs, dans la présente demande, la phase aqueuse et la phase huileuse sont imprégnées sur le support simultanément ou séparément, et le support en polyuréthanne est l'unique support d'imprégnation.

Il est connu par le document WO-A-2004/006879, un produit pour l'hygiène, qui comprend un applicateur sur lequel une phase lipidique et une phase aqueuse ont été appliquées. Toutefois, la phase lipidique est solide ou semi-solide à température ambiante, et elle comprend généralement une cire, ce qui serait rédhibitoire dans la présente invention, car la présence d'un composé solide ou semi-solide à température ambiante dans la composition revendiquée selon la présente invention diminuerait fortement, voire annulerait l'efficacité de démaquillage de l'article car ces composés, de par leur état solide à semi solide, ne sont pas des solvants efficaces pour dissoudre et éliminer les émulsions huileuses et autres compositions cireuses dont sont constitués les produits de maquillage. En outre, dans ce document, l'utilisation de mousse de polyuréthanne est limitée à l'imprégnation d'une phase lipidique, la phase aqueuse étant imprégnée sur une éponge de cellulose.

L'article revendiqué dans la présente demande présente l'avantage d'avoir une composition d'imprégnation qui reste stable et identique, même s'il y a un empilement d'articles, comme le montrent les résultats présentés avec les exemples.

En outre, l'article selon l'invention présente l'avantage d'être très facile à manipuler, car on l'utilise directement sur la peau, sans avoir à le mouiller, et on l'applique par simple essuyage sur la peau comme une lingette démaquillante ou un coton à démaquiller.

Lors de l'application sur la peau, la pression exercée par la main de l'utilisateur sur l'article lors de l'essuyage, fait passer la composition de l'intérieur de la mousse vers l'extérieur, et le passage de la composition biphase à travers les cellules de la mousse provoque un effet de cisaillement qui la transforme en une fine dispersion de gouttelettes d'huile dans la phase aqueuse.

L'article selon l'invention est en particulier un article cosmétique, approprié pour le nettoyage ou le démaquillage de la peau du visage et/ou du corps, des lèvres et/ou des yeux. Il peut notamment constituer une lingette, une compresse, une éponge, un tampon (« pad » en Anglais). Il peut avoir toute forme appropriée pour une utilisation pratique sur le visage ou le corps.

L'invention a aussi pour objet l'utilisation cosmétique de l'article tel que défini ci-dessus, pour le nettoyage et/ou le démaquillage de la peau, des lèvres et/ou des yeux.

L'invention a encore pour objet un procédé cosmétique de nettoyage et/ou de démaquillage de la peau, des lèvres et/ou des yeux, consistant à passer sur la peau, des lèvres et/ou les yeux, un article tel que défini ci-dessus.

La composition utilisée selon l'invention pour l'imprégnation du support étant destinée à une application topique, elle contient un milieu physiologiquement acceptable, c'est-à-dire compatible avec la peau, les muqueuses (lèvres), les cheveux et le cuir chevelu.

### Support

Le support est une mousse hydrophile de polyuréthanne. Par « hydrophile », on entend une mousse qui absorbe l'eau. De manière préférée, la mousse utilisée selon l'invention a un pouvoir maximal d'absorption d'eau de 0,4 à 3,5 g/cm³ et un pouvoir de rétention d'eau de 0,07 à 2 g/cm³.

Le pouvoir d'absorption d'eau correspond à la quantité maximale d'eau (en grammes) pouvant être absorbée sur un volume de mousse sèche (en cm³). Cette quantité est déterminée en pesant une mousse de volume déterminée avant imprégnation, puis en immergeant la mousse dans de l'eau, la mousse étant ainsi saturée en eau quand on la ressort, et à peser la mousse imprégnée. On peut aussi simplement déterminer le volume d'eau avant et après immersion de la mousse, la différence correspondant au poids d'eau absorbée par le volume de mousse.

Le pouvoir de rétention d'eau correspond à la quantité d'eau (en grammes) qui reste dans la mousse par rapport au volume de mousse sèche (en cm³), après application sur la mousse imprégnée à sa capacité maximale d'imprégnation, d'une force de 80 Newtons pendant une seconde.

Par ailleurs, cette mousse est élastique. Par « élastique », on entend une mousse ayant une élasticité allant de 10 % à 500 % d'élongation, de préférence de 50 % à 150 % d'élongation.

Le support est de préférence en mousse de polyuréthanne, telle que décrite par exemple dans le document DE10327707. Ce sont des mousses de polyuréthanne à cellules ouvertes, et de préférence à fines pores. La mousse de polyuréthanne peut être obtenue selon les techniques classiques de préparation des polyuréthannes, par exemple par préparation d'un prépolymère de polyuréthanne ayant des groupes isocyanates libres, ce prépolymère étant obtenu à partir d'un diisocyanate et d'un polyol ou d'un polyester ou d'un polyol polyéther, puis hydrolyse du prépolymère au contact de l'eau ou réaction avec un composé aminé, en présence de catalyseurs appropriés. On peut aussi utiliser tout autre procédé de préparation connu de l'homme de l'art.

La mousse constituant le support peut avoir une épaisseur allant par exemple de 0,5 à 10 mm et mieux de 1 à 5 mm.

En outre, ce support peut avoir toute taille et toute forme appropriées au but recherché. Il peut être par exemple de forme rectangulaire, ronde, carrée ou ovale. Il a généralement une surface comprise entre 0,005 m² et 0,1 m², de préférence entre 0,01 m² et 0,05 m².

Comme mousse, on peut utiliser par exemple la mousse vendue par la société Dicon, d'une épaisseur de 3 mm ; ou celle vendue par la société Martini, d'une épaisseur de 3 mm ; ou celle vendue par Otto Bock sous le nom PU-Schwamm, d'une épaisseur de 5 mm ; ou celle vendue par Rynel Medical Foam, d'une épaisseur de 5 mm.

Le taux d'imprégnation de la composition sur le support va généralement de 10 à 1500 % en poids par rapport au poids de support, de préférence de 50 à 1000 % et encore mieux entre 70 et 500 %.

Les techniques d'imprégnation des supports par les compositions utilisées selon l'invention sont bien connues dans ce domaine et sont toutes applicables à la présente invention. Ainsi, la composition biphase peut être ajoutée sur le support par exemple par immersion, enduction, pulvérisation, etc. Elle peut être d'abord préparée sous forme de biphase par agitation de la phase huileuse et de la phase aqueuse, puis imprégnée sur le support, ou bien on peut ajouter sur le support l'une après l'autre, l'une des phases puis l'autre phase, le sens d'introduction des phases n'étant pas déterminant. Selon un mode préféré de réalisation de l'invention, on imprègne le support par introduction simultanée des deux phases de la composition biphase sous agitation soit par une seule arrivée, pour assurer une imprégnation homogène des deux phases, soit simultanément mais de manière indépendante par deux arrivées distinctes.

### Composition biphase

La composition d'imprégnation selon l'invention comprend une phase aqueuse et une phase huileuse distinctes. Ces deux phases sont distinctes, c'est-à-dire qu'elles sont visibles l'une au-dessus de l'autre au repos et ne forment une dispersion qu'après agitation du mélange et qu'elles se séparent en deux phases quand l'agitation cesse.

Le rapport pondéral entre la phase aqueuse et la phase huileuse dans la composition de l'invention va de 25/75 à 90/10, de préférence 30/70 à 70/30, mieux de 40/60 à 60/40 et encore mieux 50/50. Ainsi, la phase aqueuse représente généralement de 25 à 90 % en poids, de préférence de 30 à 70 % en poids et mieux de 40 à 60 % en poids par rapport au poids total de la composition.

Selon un mode préféré de réalisation de l'invention, la composition destinée à imprégner un support présente une viscosité de préférence inférieure à 150 mPa.s et plus préférentiellement inférieure à 100 mPa.s. Cette viscosité va de préférence de 1 mPa.s à 100 mPa.s, mesurée à la température ambiante (environ 25°C) avec un appareil RHEOMAT RM 180, mobile 1.

### Phase aqueuse

La phase aqueuse de la composition d'imprégnation utilisée selon l'invention comprend de l'eau et tout additif hydrosoluble ou hydrodispersible. L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, l'eau d'Eaux Bonnes, l'eau de Rochefort, l'eau de Saint Christau, l'eau des Fumades et l'eau de Tercis-les-bains, l'eau d'Avene. La phase aqueuse peut comprendre aussi de l'eau thermale reconstituée, c'est-à-dire une eau contenant des oligoéléments tels que zinc, cuivre, magnésium, etc.., reconstituant les caractéristiques d'une eau thermale.

Comme additifs hydrosolubles, on peut citer notamment les polyols tels que la glycérine et les glycols comme l'hexylène glycol, les polyéthylèneglycols et le polypropylèneglycol. Les polyols peuvent être présents en une quantité allant par exemple de 0 à 5 % en poids, de préférence de 0,01 et 5 %, mieux de 0,05 à 3 % en poids et encore mieux de 0,1 à 3 % en poids par rapport au poids total de la composition. Selon un mode préféré de réalisation de l'invention, la composition contient au moins un polyol, de préférence la glycérine ou l'hexylène glycol ou leurs mélanges.

Comme additifs hydrosolubles, on peut citer aussi les alcools primaires en C₂-C₈, et notamment l'éthanol. Selon un mode particulier de réalisation de l'invention, la composition est de préférence pratiquement exempte d'éthanol. On entend ici par "pratiquement exempte d'éthanol", une composition contenant moins de 2 % en poids et de préférence moins de 1 % en poids d'éthanol par rapport au poids total de la composition.

### Phase huileuse

Les constituants de la phase huileuse ont un point de fusion inférieur à 25°C, et la phase huileuse a donc un point de fusion inférieur à 25°C, c'est-à-dire qu'elle est liquide à température ambiante (25°C).

La phase huileuse représente généralement de 10 à 75 %, de préférence de 30 à 70 % en poids, et mieux de 40 à 60 % en poids par rapport au poids total de la composition.

La phase huileuse de la composition selon l'invention peut être constituée d'une ou plusieurs huiles, celles-ci pouvant être des huiles minérales, végétales ou synthétiques ou encore des huiles de silicone. Elle peut comprendre en outre des additifs liposolubles ou lipodispersibles. Toutefois, elle est exempte de composé ayant un point de fusion supérieur à 25°C, et notamment de cire.

Selon un mode préféré de réalisation de l'invention, la phase huileuse comprend une ou plusieurs huiles choisies parmi les huiles hydrocarbonées d'origine minérale ou synthétique et les huiles de silicone. Plus particulièrement, la phase huileuse contient avantageusement une ou plusieurs huiles volatiles choisies parmi les huiles hydrocarbonées volatiles d'origine minérale ou synthétique et les huiles de silicone volatiles.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par "huile volatile", on entend une huile susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

Comme huiles hydrocarbonées volatiles d'origine minérale ou synthétique, on peut citer les iso-alcanes (appelées aussi isoparaffines) en C₈-C₁₆, tels que l'isododécane, l'isodécane, l'isohexadécane, comme par exemple les iso-alcanes vendus sous les noms commerciaux lsopar par la société Exxon Chemical ou les huiles vendues sous les noms commerciaux Permethyl par la société Presperse ; et leurs mélanges.

Comme huiles hydrocarbonées non-volatiles d'origine minérale ou synthétique, on peut citer l'huile minérale (Mineral OIL en nom INCI), l'huile de vaseline, le polyisobutène hydrogéné tel que l'huile de Parléam® ; et leurs mélanges.

Par huile de silicone, on entend une huile contenant au moins un atome de silicium, et notamment contenant des groupes Si-O. L'huile de silicone peut être choisie parmi les huiles de silicone non volatiles, les huiles de silicone volatiles et leurs mélanges.

Les huiles de silicone volatiles utilisables dans l'invention peuvent être choisies notamment parmi les huiles de silicone ayant un point éclair allant de 40°C à 102°C, de préférence ayant un point éclair supérieur à 55°C et inférieur ou égal à 95°C, et préférentiellement allant de 65°C à 95°C. Comme huiles de silicone volatiles, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemples d'huiles de silicone volatiles, on peut citer notamment les cyclopolydiméthylsiloxanes (nom INCI : cyclomethicone), telles que le cyclopentasiloxane, le cyclohexasiloxane, l'octaméthylcyclotétrasiloxane, le décaméthylcyclopentasiloxane, le dodécaméthylcyclohexasiloxane ; les silicones linéaires telles que l'heptaméthylhexyl-trisiloxane, l'heptaméthyloctyl-trisiloxane, l'hexaméthyl-disiloxane, l'octaméthyl-trisiloxane, le décaméthyltétrasiloxane, le dodécaméthyl pentasiloxane ; et leurs mélanges.

Les huiles de silicone non volatiles utilisables dans l'invention peuvent être choisies parmi les polydiméthylsiloxanes (PDMS), et les polyméthylsiloxanes phénylés tels que les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; les polysiloxanes modifiés par des acides gras, des alcools gras ou des polyoxyalkylènes, et leurs mélanges.

Selon un mode particulier de réalisation de l'invention, la phase huileuse contient au moins une huile volatile et notamment au moins un isoalcane choisi parmi l'isododécane, l'isohexadécane et leurs mélanges. Selon un autre mode préférentiel de réalisation de l'invention, la phase huileuse contient au moins un isoalcane et au moins une huile de silicone volatile.

Selon un autre mode particulier de réalisation de l'invention, la phase huileuse contient au moins une huile minérale (Mineral oil), notamment une huile minérale (Mineral oil) et une huile de silicone non volatile (dimethicone).

Par ailleurs, la phase huileuse peut contenir en outre une ou plusieurs autres huiles volatiles ou non, choisies parmi les huiles hydrocarbonées d'origine animale ou végétale, les esters et éthers de synthèse, les alcools gras, les huiles fluorées et leurs mélanges.

Par huile fluorée, on entend une huile contenant au moins un atome de fluor.

On peut citer par exemple comme huiles utilisables dans la composition de l'invention :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'amande d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle.

Les huiles peuvent éventuellement être constituées uniquement d'huiles volatiles. Le conditionnement de la pile de mousse sera alors choisi en conséquence pour en garantir une bonne étanchéité.

### Tensioactifs

La composition biphase selon l'invention peut comprendre éventuellement un ou plusieurs tensioactifs dans l'une ou l'autre des phases, notamment quand elle est utilisée comme composition de démaquillage ou de nettoyage, car la présence d'un tensioactif permet à la fois d'obtenir un bon démaquillage des compositions de maquillage et notamment des mascaras, et aussi d'avoir une absence de sensation grasse lors du démaquillage. Toutefois, la composition de l'invention peut aussi être exempte de tensioactif, et si elle en contient, la quantité de tensioactif doit être telle que la composition reste au repos sous forme de deux phases distinctes et non pas sous forme d'émulsion.

Ainsi, la quantité de tensioactif(s) en matière active doit être en une quantité telle que les deux phases restent distinctes au repos et ne se mélangent pas pour former une émulsion. Cette quantité doit généralement être inférieure ou égale à 1,5 % en poids par rapport au poids total de la composition. Elle peut aller par exemple de 0,001 à 1,5 % en poids, de préférence de 0,002 à 1 % en poids et mieux de 0,01 à 0,5 % en poids par rapport au poids total de la composition.

Les tensioactifs peuvent être choisis parmi les tensioactifs non ioniques, anioniques, zwitterioniques ou amphotères, et leurs mélanges. Selon un mode préféré de réalisation de l'invention, le tensioactif s'il est présent est de préférence non ionique.

Parmi les agents tensioactifs non ioniques, ceux particulièrement préférés sont :
- les esters gras de sorbitol polyoxyéthylénés tels que le produit vendu sous la dénomination TWEEN 20 par la Société ICI.
- les alcools gras polyoxyéthylénés tels que le produit vendu sous la dénomination REMCOPAL 21912 AL par la Société GERLAND.
- les alkylphénols polyoxyéthylénés tels que le produit vendu sous la dénomination TRITON X 100 par la Société RÔHM-HAAS, et
- les condensats d'oxyde d'éthylène et d'oxyde de propylène tels que ceux vendus sous les dénominations SYNPERONIC PE par la Société ICI et en particulier ceux référencés L 31, L 64, F 38, F 88, L 92, P 103, F 108 et F 127 (nom CTFA : Poloxamer).
- les alkylpolyglycosides tels que ceux de formule générale (I) suivante :

   R-O-(G)ₓ (I)

   dans laquelle R représente un radical alkyle linéaire ou ramifié, saturé ou insaturé, comportant de 6 à 30 atomes de carbone, G représente un sucre réduit comportant de 5 à 6 atomes de carbone, et x désigne une valeur allant de 1 à 15. Comme alkylpolyglucosides, on peut citer par exemple le decylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP et PLANTACARE 2000 UP par la société Cognis, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Cognis ; et le coco-glucoside comme le produit commercialisé sous la dénomination PLANTACARE 818/UP par la société Cognis, et leurs mélanges.
- les diméthicone copolyols ou les mélanges les contenant tels que le produit vendu sous la dénomination DC 5225C par la société Dow Corning.
- et leurs mélanges.

Parmi les agents tensioactifs anioniques, on peut notamment citer :
- les alkylsulfates, les alkyl éther sulfates et leurs sels, notamment leurs sels de sodium, comme le mélange de Sodium Laureth Sulfate / Magnesium Laureth Sulfate / Sodium Laureth-8 Sulfate / Magnesium Laureth-8 Sulfate, vendu sous le nom de Texapon ASV par la société Cognis ; le lauryl éther sulfate de sodium (nom CTFA : sodium laureth sulfate) comme celui commercialisé sous les dénominations TEXAPON N 40 et TEXAPON AOS 225 UP par la société Cognis ; le lauryl sulfate d'ammonium (nom CFTA : Ammonium lauryl sulfate) tel que le produit commercialisé par la société Huntsman sous la dénomination EMPICOL AL 30FL ; l'alkyl (C12-C14) éther (9 OE) sulfate d'ammonium commercialisé sous la dénomination RHODAPEX AB/20 par la société Rhodia Chimie ; le lauryl sulfate de triéthanolamine (nom CTFA : TEA-lauryl sulfate) tel que le produit commercialisé par la société Huntsman sous la dénomination EMPICOL TL40 FL ou celui commercialisé par la société Cognis sur la dénomination TEXAPON T42 ;
- les alkylsulfoacétates tels que celui vendu sous la dénomination LATHANOL LAL par la Société STEPAN ;
- les sulfosuccinates d'alkyle, par exemple le mono-sulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hemi-sulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Cognis, le mono-sulfosuccinate d'amide laurique oxyéthyléné (5 OE) commercialisé sous la dénomination LEBON A-5000 par la société Sanyo, le sel di-sodique de mono-sulfosuccinate de lauryl citrate oxyéthyléné (10 OE) commercialisé sous la dénomination REWOPOL SB CS 50 par la société Witco, le sel disodique de mono-sulfosuccinate de mono-éthanolamide ricinoléique commercialisé sous la dénomination REWODERM S 1333 par la société Witco ;
- les polypeptides qui sont obtenus par exemple par condensation d'une chaîne grasse sur les aminoacides de céréale et notamment du blé et de l'avoine, comme par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl amino-acides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic ;
- Les dérivés des aminoacides, par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-methylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-methylalanine triéthanolamine, commercialisé sous la dénomination ALANONE ALTA par la société Kawaken ; les N-acylglutamates comme le mono-cocoylglutamate de triéthanolamine commercialisé sous la dénomination ACYLGLUTAMATE CT-12 par la société Ajinomoto, et le lauroyl-glutamate de triéthanolamine commercialisé sous la dénomination ACYLGLUTAMATE LT-12 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les dérivés de la glycine, comme le N-cocoylglycinate de sodium et le N-cocoylglycinate de potassium tels que les produits commercialisés sous les dénominations AMILITE GCS-12 et AMILITE GCK-12 par la société Ajinomoto ;
- les sulfonates, par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco ou commercialisé sous la dénomination BIO-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro ;
- les iséthionates, notamment les acyliséthionates comme le cocoyl-iséthionate de sodium, tel que le produit commercialisé sous la dénomination JORDAPON CI P par la société Jordan.

Parmi les tensioactifs amphotères ou zwitterioniques, on peut notamment citer :
- les dérivés alkylamido alkylamines tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl amidoéthyl, N-hydroxyéthyl glycinate de sodium (nom CTFA : sodium cocoamphoacetate) et le mélange d'éthanolamides d'acide de coco (nom CTFA : Cocamide DEA) ;
- les bétaïnes, comme par exemple la cocobétaïne comme le produit commercialisé sous la dénomination DEHYTON AB-30 par la société Cognis, la laurylbétaïne comme le produit commercialisé sous la dénomination GENAGEN KB par la société Clariant, la laurylbétaïne oxyethylénée (10 OE), comme le produit commercialisé sous la dénomination LAURYLETHER(10 OE)BETAINE par la société Shin Nihon Rica, la stéarylbétaïne oxyéthylénée (10 OE) comme le produit commercialisé sous la dénomination STEARYLETHER(10 OE)BETAINE par la société Shin Nihon Rica ;
- les alkylamidopropylbétaïnes et leurs dérivés comme par exemple la cocamidopropyl bétaine commercialisée sous la dénomination LEBON 2000 HG par la société Sanyo, ou commercialisée sous la dénomination EMPIGEN BB par la société Albright & Wilson ou sous la dénomination TEGO BETAINE par la Société GOLDSCHMIDT , la lauramidopropyl bétaïne commercialisée sous la dénomination REWOTERIC AMB12P par la société Witco ;
- les dérivés d'imidazoline tels que le produit vendu sous la dénomination CHIMEXANE HD par la Société CHIMEX, et
- leurs mélanges.

### Agent de déphasage

La composition biphase peut contenir en outre un ou plusieurs agents favorisant le déphasage en deux phases après agitation afin de réduire le temps de déphasage. Comme agents de déphasage, on peut citer par exemple les chlorures d'alkyldiméthylbenzylammonium comme décrit dans le document EP-A-0603080, les esters d'acide diméthylcarboxylique et d'alcane diol, le bicarbonate de sodium, les alkylglucosides alkoxylés à ammonium quaternaire comme décrit dans le document EP-A-0847746, les copolymères de PVP comme décrit dans le document EP-A-0996408, un mélange de polydextrose et sucrose comme décrit dans le document US-A-6,727,209. Ces agents peuvent être présents en une quantité allant par exemple de 0,005 à 5 %, de préférence de 0,01 à 4 % et mieux de 0,5 à 4 % du poids total de la composition.

### Adjuvants

Outre ceux indiqués ci-dessus, la composition biphase utilisée dans l'article selon l'invention peut également contenir des adjuvants ou additifs cosmétiques conventionnels qui se trouveront dans l'une ou l'autre phase selon leur nature hydrophile ou lipophile, tels que par exemple des parfums, des colorants, des agents conservateurs et bactéricides, des agents adoucissants, des tampons, des humectants, des filtres U.V. (ou filtres solaires), des électrolytes tel que le chlorure de sodium comme indiqué plus haut, ou un ajusteur de pH (par exemple acide citrique ou hydroxyde de sodium), et leurs mélanges.

Comme conservateurs, on peut utiliser tout conservateur habituellement utilisé dans les domaines considérés, tels que par exemple les parabens, le gluconate de chlorhexidine, le chlorhydrate de polyhexaméthylène biguanide (nom CTFA Polyaminopropyl biguanide) et leurs mélanges. Selon un mode préféré de réalisation de l'invention, la composition contient du chlorhydrate de polyhexaméthylène biguanide, seul ou en mélange avec d'autres conservateurs.

Comme bactéricide, on peut par exemple utiliser un mono(C₃-C₉)alkyl-ou (C₃₋C₉)alkényléther de glycérol dont la fabrication est décrite dans la littérature, en particulier dans E. Baer, H.O.L. Fischer - J. Biol. Chem. 1941, 140, p.397. Parmi ces mono(C₃-C₉)alkyl-ou mono-(C₃-C₉)alkényléthers de glycérol, on utilise de préférence le 3-[(2-ethylhexyl)oxy]-1,2-propanediol, le 3-[(heptyl)oxy]-1,2-propanediol, le 3-[(octyl)oxy]-1,2-propanediol et le 3-[(allyl)oxy]-1,2-propanediol. Un mono(C₃-C₉)alkyléther de glycérol plus particulièrement préféré selon la présente invention est le 3-[(2-ethylhexyl)oxy]-1,2-propanediol, vendu par la société SCHULKE & MAYR G.m.b.H. sous la dénomination commerciale SENSIVA SC 50 (nom INCI : Ethylhexylglycerin).

Parmi les agents adoucissants, on peut en particulier citer l'allantoïne, le bisabollol, les planctons, et certains extraits de plantes comme les extraits de rose et les extraits de mélilot.

Le ou les actifs pouvant être présents dépendent du but final de la composition. Comme actifs utilisables dans la composition de l'invention, on peut citer par exemple, les enzymes (par exemple lactoperoxydase, lipase, protéase, phospholipase, cellulases) ; les agents hydratants tels que les hydrolysats de protéines ; le hyaluronate de sodium ; les anti-inflammatoires , les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les huiles essentielles ; et leurs mélanges ; et tout actif approprié pour le but final de la composition.

Les adjuvants lipophiles sont dissous directement dans les huiles, tandis que les adjuvants hydrophiles sont ajoutés dans la phase aqueuse ou sont dispersés dans la composition à l'aide des tensioactifs présents.

### Test de stabilité des articles

### 1) composition utilisée

Pour les tests comparatifs de stabilité des piles d'articles, on a utilisé la composition biphase suivante, où les pourcentages sont des pourcentages en poids :

### Phase huileuse

- Mineral oil 50 %
- Dimethicone 50 %

### Phase aqueuse

- Hexylene glycol 0,5 %
- PEG-60 Hydrogenated Castor Oil 0,2 %
- Decyl glucoside 0,2 %
- parfum 0,017 %
- Disodium EDTA (chélatant) 0,2 %
- conservateur 1,8 %
- Chlorure de sodium 0,5 %
- Eau déminéralisée 96,583 %

Mode opératoire : On mélange les constituants de la phase huileuse d'une part et ceux de la phase aqueuse d'autre part. Puis on mélange les deux phases. On obtient une composition qui, au repos, comporte une phase aqueuse et une phase huileuse distinctes. Par agitation, les deux phases donnent une émulsion qui se sépare de nouveau en deux phases au repos.

### 2) Technique de détermination de la sédimentation des fractions huileuse et aqueuse au sein d'une pile d'articles après un temps de conservation. (à utiliser quand la phase huileuse ne contient pas d'huile volatile)

Avant d'effectuer ce test, il faut, pour un support donné et une composition donnée, (1) vérifier que le support ne contient pas d'eau ou de solvant résiduels après l'avoir laissé 72 heures à 45°C ; (2) vérifier que toute la quantité d'eau est évaporée quand ce support imprégné de 500% de phase aqueuse est entreposé pendant 72 heures à 45°C, et, sinon, en déduire la fraction résiduelle de phase aqueuse sur le support, et (3) vérifier que toute la phase huileuse est conservée sur le support quand ce support imprégné de 500% de phase huileuse est entreposé pendant 72 heures à 45°C.

Dans le cas des supports utilisés dans les exemples de l'invention et dans l'exemple comparatif, il a été constaté (1) qu'il n'y avait pas de perte de poids du support après 72 H à 45°C, ce qui signifie que ces supports ne contiennent ni eau résiduelle ni solvant résiduel, (2) qu'il restait une quantité résiduelle de phase aqueuse du support imprégné de phase aqueuse, quantité dont il est tenu compte dans le calcul présenté ci-dessous, et (3) qu'il n'y avait pas de perte de phase huileuse, ce qui est logique puisque la composition d'imprégnation ci-dessus ne contient pas d'huile volatile.

Pour déterminer les fractions aqueuse et huileuse dans les articles, on pèse l'article, on le place à 45°C à l'étuve pendant 24 heures, et on le repèse à sa sortie de l'étuve.

Dans le cas des mousses, chaque support imprégné est pris séparément. Dans le cas des disques de non-tissé, utilisés pour l'exemple comparatif, ces non-tissés étant plus légers, ils sont beaucoup plus nombreux dans une pile que les disques de mousse de polyuréthanne pour une boîte de dimension identique. Ainsi, pour les pesées et les calculs de sédimentation, les disques de non-tissé, au nombre de 60 dans chaque boîte, sont pesés par 5 (c'est-à-dire 12 pesées pour une pile de 60 disques de non-tissé), alors que les disques de mousse de polyuréthanne, au nombre de 15 dans chaque boîte, sont pesés individuellement (c'est-à-dire 15 pesées pour une pile de 15 disques de mousse).

La fraction aqueuse imprégnée se calcule de la manière suivante : M phase aqueuse = M imprégnée (avant passage à l'étuve) - M imprégnée (après 24h à 45°C) + M fraction résiduelle de phase aqueuse sur le support

La fraction huileuse imprégnée se calcule de la manière suivante : M phase huileuse = M imprégnée (après 24h à 45°C) - M non imprégnée - M fraction résiduelle de phase aqueuse sur le support.

M imprégnée est le poids du support imprégné de la composition
M non imprégnée est le poids du support avant imprégnation
M fraction résiduelle de phase aqueuse sur le support est la masse de phase aqueuse restant piégée dans la mousse (=quantité résiduelle de phase aqueuse du support imprégné de phase aqueuse)

Cette méthode permet donc de déterminer précisément les quantités de phases aqueuse et huileuse présentes sur un support tel que la mousse de polyuréthanne hydrophile utilisée dans l'exemple selon l'invention ci-après et tel que le non-tissé utilisé dans l'exemple comparatif ci-dessous.

Avant d'utiliser cette méthode pour un autre support, il est recommandé de vérifier, par un simple test à l'étuve, leur comportement en présence d'eau, d'huile et du mélange. Comme indiqué ci-dessus, dans le cas de la mousse donnée en exemple, une fraction d'eau non volatile devra être retranchée ou ajoutée.

### 3) Exemple comparatif avec un support en non-tissé :

60 feuilles rondes (diamètre = 57 mm) de non-tissé 100% coton (grammage = 100 +/- 5 g/m²) ont été imprégnées avec la composition biphase indiquée ci-dessus, à un taux d'imprégnation de 500 % (5 g de démaquillant pour 1 g de non-tissé), c'est-à-dire en introduisant 250 % de phase aqueuse et 250 % de phase huileuse sur chaque feuille de non-tissé (2,5 g de phase aqueuse et 2,5 g de phase huileuse pour 1 g de non-tissé).

Pour ce support non-tissé, il n'y a pas d'eau résiduelle, c'est-à-dire pas de quantité résiduelle de phase aqueuse du support imprégné de phase aqueuse, dont il faudrait tenir compte dans le calcul.

La hauteur de la pile de disques de non-tissé secs est de 4,8 cm. Les feuilles sont conditionnées dans une boîte plastique du type « boîte de tampons à démaquiller ou pads ».

Après un temps de conservation de deux semaines, on mesure la nouvelle répartition du jus dans la pile de disques de non-tissé (tableau 1).

**Tableau 1**

| Feuille de non-tissé | Poids de support | Résultats au temps zéro | | Résultats après 15 jours | |
|---|---|---|---|---|---|
| | Poids (M) de non-tissé sec (g) | Poids (M) de phase aqueuse (g) | Poids (M) de phase huileuse (g) | Poids (M) de phase aqueuse (g) | Poids (M) de phase huileuse (g) |
| 5 du haut | 1.25 | 3.16 | 3.19 | 2.75 | 3.26 |
| 5 suivantes | 1.32 | 3.3 | 3.31 | 3.12 | 3.33 |
| 5 suivantes | 1.33 | 3.32 | 3.34 | 3.2 | 3.36 |
| 5 suivantes | 1.38 | 3.47 | 3.43 | 3.33 | 3.44 |
| 5 suivantes | 1.39 | 3.49 | 3.49 | 3.31 | 3.51 |
| 5 suivantes | 1.36 | 3.42 | 3.48 | 3.26 | 3.47 |
| 5 suivantes | 1.35 | 3.38 | 3.49 | 3.28 | 3.51 |
| 5 suivantes | 1.31 | 3.28 | 3.28 | 3.32 | 3.12 |
| 5 suivantes | 1.34 | 3.35 | 3.36 | 3.46 | 3.29 |
| 5 suivantes | 1.34 | 3.35 | 3.35 | 3.51 | 3.26 |
| 5 suivantes | 1.26 | 3.17 | 3.20 | 3.32 | 3.12 |
| 5 du bas | 1.35 | 3.38 | 3.39 | 3.60 | 3.30 |

Ce tableau montre que, après 15 jours, les proportions de phase aqueuse et de phase huileuse ne sont plus les mêmes dans les différents articles selon leur position dans la pile d'articles et que les articles en bas de la pile contiennent beaucoup plus de phase aqueuse que ceux en haut de la pile.

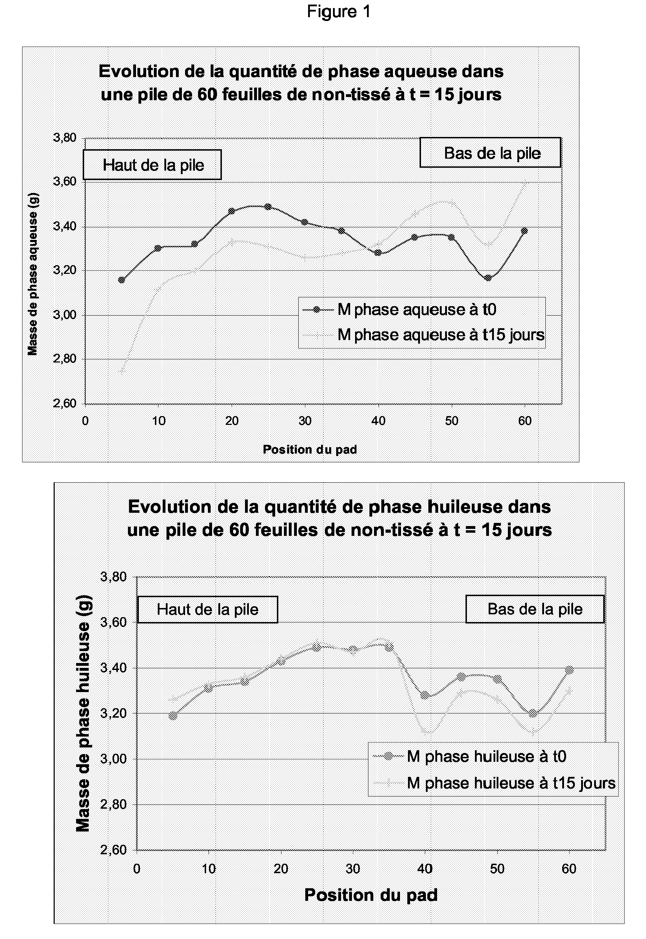

Les graphes 1 et 2 de la figure 1 illustrent le phénomène observé pour chaque phase.

### 4) Exemple 1 selon l'invention

15 supports ronds en mousse de polyuréthanne hydrophile (Martini, épaisseur 3 mm, diamètre 50 mm) ont été imprégnés à 500 % de la composition indiquée ci-dessus, soit 250 % de phase aqueuse et 250 % de phase huileuse. La hauteur de cette pile de 15 disques de mousse secs est de 4,8 cm. Les mousses sont conditionnées dans une boîte plastique du type « boîte de tampons à démaquiller ».

Après 2 mois de conservation, on détermine les quantités de phase aqueuse et de phase huileuse présentes dans chaque mousse. Les résultats sont représentés dans le tableau 2.

**Tableau 2**

| Mousse | Poids de support | Résultats au temps zéro | | Résultats après 2 mois | |
|---|---|---|---|---|---|
| | Poids (M) de mousse sèche (g) | Poids (M) de phase aqueuse (g) | Poids (M) de phase huileuse (g) | Poids (M) de phase aqueuse (g) | Poids (M) de phase huileuse (g) |
| Haut | 0.56 | 1.45 | 1.37 | 1.43 | 1.44 |
| Suivante | 0.58 | 1.51 | 1.41 | 1.33 | 1.35 |
| Suivante | 0.59 | 1.53 | 1.43 | 1.51 | 1.48 |
| Suivante | 0.55 | 1.41 | 1.36 | 1.37 | 1.39 |
| Suivante | 0.58 | 1.48 | 1.45 | 1.46 | 1.43 |
| Suivante | 0.60 | 1.51 | 1.49 | 1.38 | 1.46 |
| Suivante | 0.62 | 1.56 | 1.54 | 1.61 | 1.62 |
| Suivante | 0.59 | 1.50 | 1.46 | 1.40 | 1.42 |
| Suivante | 0.58 | 1.46 | 1.46 | 1.38 | 1.41 |
| Suivante | 0.58 | 1.51 | 1.41 | 1.40 | 1.42 |
| Suivante | 0.59 | 1.49 | 1.49 | 1.39 | 1.48 |
| Suivante | 0.63 | 1.59 | 1.59 | 1.59 | 1.62 |
| Suivante | 0.58 | 1.46 | 1.49 | 1.37 | 1.44 |
| Suivante | 0.51 | 1.29 | 1.25 | 1.36 | 1.27 |
| Bas | 0.60 | 1.51 | 1.49 | 1.59 | 1.44 |

Ce tableau montre que, après 2 mois, les proportions de phase aqueuse et de phase huileuse restent pratiquement les mêmes dans les articles de la pile et que les articles en bas de la pile contiennent une quantité de phase aqueuse qui varient peu par rapport à celles d'origine. Pour l'article du haut, la perte en phase aqueuse est de 1,38 % alors qu'elle est de 12,97 % sur le support en non-tissé, et pour l'article le plus en bas, l'augmentation de phase aqueuse est de 5,3 % alors qu'elle est de 6,5 % pour le support en non-tissé.

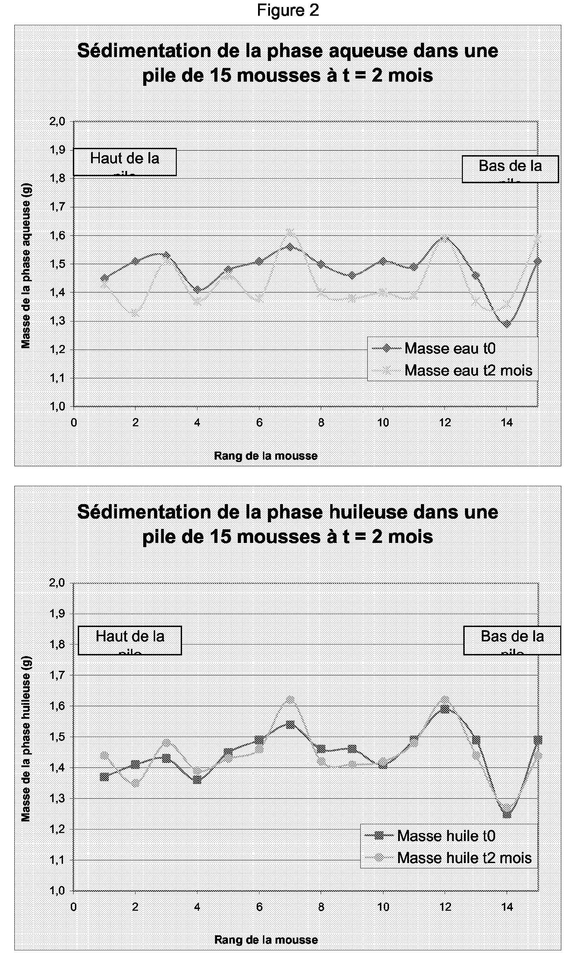

Les graphes 3 et 4 de la figure 2 illustrent le phénomène observé pour chaque phase.

### Exemples 2 à 5 selon l'invention

Les exemples ci-après de compositions selon l'invention sont donnés à titre d'illustration et sans caractère limitatif. Les noms sont en nom chimique ou en nom CTFA. Les quantités y sont données en % en poids, sauf mention contraire.

### Exemple 2 : composition démaquillante

### Phase huileuse

- Cyclopentasiloxane 14 %
- Isododécane 15 %
- Palmitate d'isopropyle 19,6 %
- parfum 0,2 %
- Ethylhexylglycerin (Sensiva SC 50) 1,2 %

### Phase aqueuse

- Glycérine 20 %
- Dipropylène glycol 12 %
- Triéthanolamine (neutralisant) 0,07 %
- Methylparaben 0,2 %
- Chlorure de sodium 0,5 %
- Disodium EDTA (chélatant) 0,08 %
- Eau déminéralisée 17,15 %

Mode opératoire : On mélange les constituants de la phase huileuse d'une part et ceux de la phase aqueuse d'autre part. Puis on mélange les deux phases.

On obtient une composition qui, au repos, comporte une phase aqueuse et une phase huileuse distinctes. Cette composition est imprégnée sur un support de mousse de polyuréthanne hydrophile MARTINI comme dans l'exemple 1. L'article obtenu peut être utilisé pour le démaquillage de la peau.

### Exemple 3

### Phase huileuse

- Cyclopentasiloxane 35 %
- Isododécane 7,5 %
- isohexadécane 7,5 %

### Phase aqueuse

- Glycérine 1,5 %
- Chlorhydrate de polyhexaméthylène biguanide 0,35 %
- Bicarbonate de sodium 1 %
- Eau déminéralisée 47,15 %

Mode opératoire : On mélange les constituants de la phase huileuse d'une part et ceux de la phase aqueuse d'autre part. Puis, on agite les deux phases.

On obtient une composition qui, au repos, comporte une phase aqueuse et une phase huileuse distinctes. Cette composition est imprégnée sur un support de mousse comme celui de l'exemple 1. L'article obtenu peut être utilisé pour le démaquillage de la peau.

### Exemple 4

### Phase huileuse

| | | |
|---|---|---|
| Cyclopentasiloxane | 27 % | |
| Isohexadécane | 19 % | |
| Ester d'acide 7,7-diméthyloctanoïque et de 1,2-propane diol | 2 % | |

### Phase aqueuse

| | |
|---|---|
| Hexylène glycol | 0,27 % |
| Parfum | 0,01 % |
| Decylglucoside (solution aqueuse à 55 %) | 0,06 % |
| Phosphate mono-potassique | 0,15 % |
| Phosphate dipotassique | 0,03 % |
| Chlorhydrate de polyhexaméthylène-biguanide (solution aqueuse à 20 %) | 0,08 % |
| Chlorure de sodium | 0,26 % |
| Eau | 49,14 % |

Mode opératoire : On mélange les constituants de la phase huileuse d'une part et ceux de la phase aqueuse d'autre part. Puis, on agite les deux phases.

On obtient une composition qui, au repos, comporte une phase aqueuse et une phase huileuse distinctes. Cette composition est imprégnée sur un support de mousse comme celui de l'exemple 1. L'article obtenu peut être utilisé pour le démaquillage de la peau.

### Exemple 5

### Phase huileuse

- cyclométhicone 28 %
- isohexadécane 19 %

### Phase aqueuse

- Poloxamer 184 0,05 %
- Tampon phosphate 0,15 %
- Chlorure de sodium 0,6 %
- conservateurs qs
- colorants qs
- eau déminéralisée qsp 100 %

Mode opératoire : On mélange les constituants de la phase huileuse d'une part et ceux de la phase aqueuse d'autre part. Puis, on agite les deux phases.

On obtient une composition qui, au repos, comporte une phase aqueuse et une phase huileuse distinctes. Cette composition est imprégnée sur un support de mousse comme celui de l'exemple 1. L'article obtenu peut être utilisé pour le démaquillage de la peau.

## Revendications

1. Article comportant (A) un support insoluble dans l'eau, constitué d'une mousse hydrophile de polyuréthanne, et (B) une composition cosmétique imprégnée sur le support et constituée d'une phase aqueuse et d'une phase huileuse distinctes en un rapport pondéral allant de 25/75 à 90/10, les constituants de la phase huileuse ayant un point de fusion inférieur à 25°C.

2. Article selon la revendication 1, **caractérisé en ce que** le support est une mousse de polyuréthanne à cellules ouvertes.

3. Article selon la revendication 1 ou 2, **caractérisé en ce que** la mousse est élastique.

4. Article selon l'une quelconque des revendications précédentes, **caractérisé en ce que** la mousse a un pouvoir maximal d'absorption d'eau de 0,4 à 3,5 g/cm³ et un pouvoir de rétention d'eau de 0,07 à 2 g/cm³.

5. Article selon la revendication 1 ou 2, **caractérisé en ce que** la mousse a une épaisseur allant de 0,5 à 10 mm.

6. Article selon l'une quelconque des revendications précédentes, **caractérisé en ce que** la mousse a une surface comprise entre 0,005 m² et 0,1 m².

7. Article l'une quelconque des revendications précédentes, **caractérisé en ce que** le taux d'imprégnation de la composition sur le support va de 10 à 1500 % en poids par rapport au poids de support.

8. Article selon l'une quelconque des revendications précédentes, **caractérisé en ce que** le rapport pondéral entre la phase aqueuse et la phase huileuse dans la composition va de 30/70 à 70/30.

9. Article selon l'une quelconque des revendications précédentes, **caractérisée en ce que** la composition comprend une quantité de tensioactif(s) allant de 0,001 à 1,5 % en poids par rapport au poids total de la composition.

10. Article selon l'une quelconque des revendications précédentes, **caractérisé en ce que** la composition présente une viscosité inférieure à 150 mPa.s.

11. Article selon l'une quelconque des revendications précédentes, **caractérisé en ce qu'**il constitue une lingette, une compresse, un tampon ou une éponge.

12. Article selon l'une quelconque des revendications 1 à 11, **caractérisé en ce qu'**il constitue un article de nettoyage et/ou de démaquillage de la peau, des lèvres et/ou des yeux.

13. Utilisation cosmétique de l'article selon l'une quelconque des revendications 1 à 11, pour le nettoyage et/ou le démaquillage de la peau, des lèvres et/ou des yeux.

14. Procédé cosmétique de nettoyage et/ou de démaquillage de la peau, des lèvres et/ou des yeux, consistant à passer sur la peau, des lèvres et/ou les yeux, un article selon l'une quelconque des revendications 1 à 11.
